# EUROPEAN PATENT SPECIFICATION

(11) **EP 3 741 327 B1**
(45) Date of publication and mention of the grant of the patent: **06.03.2024**
(21) Application number: 19175644.4
(22) Date of filing: 21.05.2019
(51) Int. Cl.: A61F 2/24, A61M 25/01

(54) **A MEDICAL ARRANGEMENT FOR INTRODUCING AN OBJECT INTO AN ANATOMICAL TARGET POSITION**
MEDIZINISCHE ANORDNUNG ZUR EINFÜHRUNG EINES OBJEKTS IN EINE ANATOMISCHE ZIELPOSITION
AGENCEMENT MÉDICAL POUR INTRODUIRE UN OBJET DANS UNE POSITION ANATOMIQUE CIBLE

(43) Date of publication of application: 25.11.2020
(73) Proprietor: HVR Cardio Oy, 02600 Espoo (FI)
(72) Inventor: KERÄNEN, Olli, 23741 Bjärred (SE)
(74) Representative: Berggren Oy

(56) References cited:
- US-A1- 2005 107 812
- US-B1- 7 981 152
- Olli Keranen: "Mountaineer with M logo 1020", Youtube, 31 October 2018 (2018-10-31), page 1 pp., XP054979803, Retrieved from the Internet: URL:https://www.youtube.com/watch?time_con tinue=100&v=770NUwJhA1A [retrieved on 2019-10-17]

## Description

### TECHNICAL FIELD OF THE INVENTION

The invention relates to a medical arrangement for introducing an object, such as an implant or medicament into an anatomical target position. The implant is a cardiac implant (like an annuloplasty medical device) and the anatomical target position an annulus of a heart valve, such as a mitral valve or tricuspid valve.

### BACKGROUND OF THE DISCLOSURE

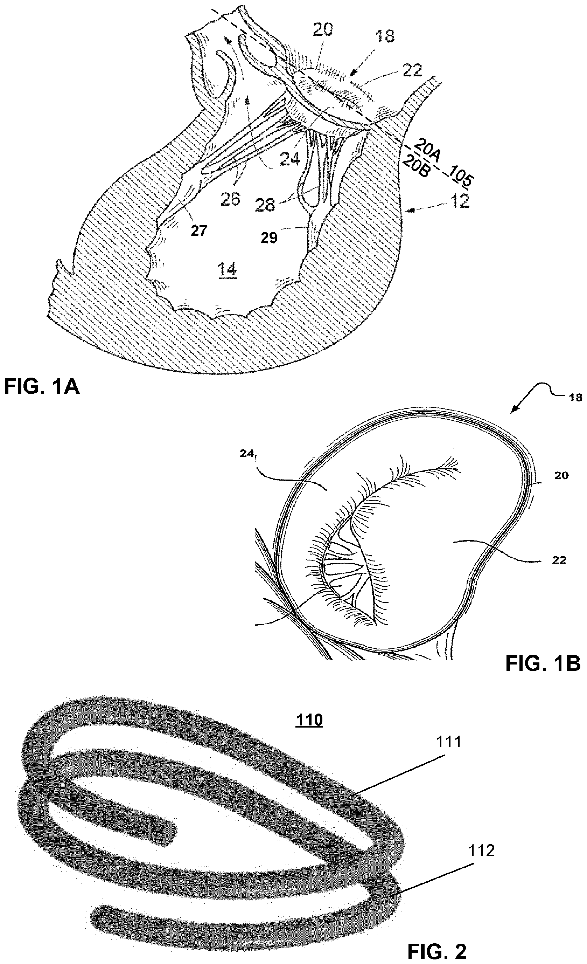

Fig. 1A illustrates a portion of the heart 12, the mitral valve 18, and the left ventricle 14 as an example of the anatomical target position. The mitral valve is at its boundary circumferenced by an annulus 20. The valve has two cusps or leaflets 22, 24. Each of these cusps or leaflets 22, 24 are connected to a respective papillary muscle 27, 29 via their respective connecting chordae 26, 28. In normal healthy individuals the free edges of the opposing leaflets will close the valve by coaptation. However, for some individuals the closure is not complete, which results in a regurgitation, also called valvular insufficiency, i.e. back flow of blood to the left atrium making the heart less effective and with potentially severe consequences for the patient. Fig. 1B illustrates a mitral valve 18, in which the leaflets 22, 24 do not close properly. This commonly occurs when the annulus 20 becomes dilated. One surgical procedure to correct this is to remove a portion of the leaflet 24 and stitch the cut edges together with one another. The procedure will pull back the annulus 20 to a more normal position. However the strength of the leaflet 24 is altered. Similar problems with a less effective heart function occur if one or both leaflets are perforated to such an extent that blood is flowing towards the left atrium, although the leaflets close properly.

In some conditions of degenerated heart function, the leaflets do not present a solid surface, as in a degenerative valve disease. The leaflet may also be ruptured, most commonly at an edge of a leaflet, resulting in an incomplete coaptation. Hence, cardiac devices and methods are developed for repairing of one or more leaflets of a heart valve, or other related anatomical structures, such as the chordae attached to the ventricular side of leaflets.

Figure 2 illustrates an exemplary implant to be delivered and introduced into an anatomical target position, and in particularly a cardiac implant 110. The implant may comprise one or more loop-shaped structures 111, 112. Advantageously one first loop-shaped structure is configured to abut a first side 20A of the heart valve and one second loop-shaped structure is configured to abut a second, opposite, side 20B of the valve to thereby trap a portion of the valve tissue 20 between the second and the first support structures 111, 112.

The implant is typically delivered via a catheter and has thus typically a delivery state, where the implant has an elongated form. In said delivery state the implant can be transferred advantageously through a catheter having diameter 7-10 mm, for example. The implant comprises typically a shape memory material having a first shape, such as the elongated form of the delivery state in a first temperature, and the second shape, such as the loop-shaped form in a second temperature. The second temperature corresponds advantageously essentially the body temperature, whereupon the implant takes the second shape, corresponding the loop-shaped form, when introduced for example with the blood flow in the atrium.

An example of prior art device is described in US 2005/107812 A1, where a cardiac valve annulus treatment device is positioned, thus enhancing treatment of the annulus. Methods generally involve advancing an anchor delivery device through vasculature of the patient to a location in the heart for treating the valve annulus, contacting the anchor delivery device with a length of the valve annulus, delivering a plurality of coupled anchors from the anchor delivery device to secure the anchors to the annulus, and drawing the anchors together to circumferentially tighten the valve annulus. Device generally includes an elongate catheter having at least one tensioning member and at least one tensioning actuator for deforming a distal portion of the catheter to help it conform to a valve annulus. The catheter device may be used to navigate a subannular space below a mitral valve to facilitate positioning of an anchor delivery device.

In addition, some problems arise due to a catheter system having both inner and outer catheters, usually steerable catheters, sometimes numerous inner catheters, where the inner catheter(s) locating inside the outer catheter limit(s) space from the implant.

It is found that the prior art cardiac implants, such as depicted above, work very well, but there are still some disadvantages relating to the introduction devices, such as catheter type devices, to deliver the implant into the anatomical target position, such as into the annulus of the heart valve. The catheter based systems are based for delivering a relative thick main catheter having a first curve portion into a first portion of the anatomical target position, such as to an atrium, then a second catheter having a second curve portion into a second portion of the anatomical target position, such as next to annulus or leaflets of the heart valve, and then possibly a third catheter having a third curve portion into a third portion of the anatomical target position, such as around the annulus of the heart valve. In some systems there might be even further catheters to with further curve portions to be delivered before the implant can be delivered and introduced into its position. The third or further catheter if used, is called as a delivery catheter. The implant is then delivered to its position inside the feeding catheters, which is delivered to its position inside the other catheters.

There are some drawbacks related to the prior art catheter based systems, such as at least the main catheter must be thick (7-10 mm or even more) so that it can carry the further catheters inside. In addition, also the delivery catheter must be relative thick so that the implant can be delivered inside the delivery catheter. When the catheters are relatively thick, it is very hard to insert the catheters into the anatomical target position. For example, a subannular space below the annulus, so between the chordae and wall or septum, is very narrow, whereupon the best channel for the thick catheters is very difficult to find and deliver, in particularly when the maneuverability and steerability of the catheters is poor. Furthermore, the surface of the inner wall is rough, having additionally numerous attachment points of chordae, which easily causes stuck of the catheters. Additional challenges arise when the catheters, especially also the delivery catheter, have memory properties or predetermined shapes, which might activate too early and thereby raising possibility to stuck the catheter into the wall or other structures of the anatomical target position, such as the heart. Thus, the time limit to insert the catheters, in particularly the delivery catheter, having memory properties, is very limited so that the catheters can be inserted into their right and accurate position before the memory property will be activated by the temperature of the anatomical target position, such as the heart.

### SUMMARY OF THE INVENTION

It is an object of the invention to alleviate and eliminate the problems relating to the known prior art. Especially the object of the invention is to provide a medical arrangement for introducing an implant or medicament into an anatomical target position in an easy, fast, safe and accurate manner with a high degree of control. In addition, the object of the invention is to minimize the sizes of the catheters used and at the same time minimize stuck of the catheters and thereby minimize stress introduced for the anatomical target position.

The object of the invention can be achieved by the features of independent claim.

The invention relates to a medical arrangement for introducing an object, such as an implant or medicament, into an anatomical target position, such as a cardiac implant into an annulus of a heart valve, according to claim 1.

A medical arrangement according to the invention is configured to introduce an object, such as a medicament or an implant, into an anatomical target position. In particularly the invention is configured to introduce a cardiac implant, or an annuloplasty medical device, from a distal end of the arrangement into an anatomical target position, such as into an annulus of a heart valve. The heart valve may be a mitral valve or tricuspid valve, for example, not limiting to those only. It is to be understood that the object can be also some other object, such as medicine, for example.

According to an example the object is the implant, which comprises in a use a loop shaped support portion, having either one or more loops or coils so that one first loop-shaped structure can be configured to abut a first side of the heart valve and one second loop-shaped structure to abut a second, opposite, side of the valve to thereby trap a portion of the valve tissue between the second and the first support structures. It is also possible that there is only the one first loop-shaped structure, which is configured to abut a first side of the heart valve, and not the second support structures, or vice versa. The implant is advantageously adapted to support for a mitral valve upon being fully delivered.

According to an embodiment the medical arrangement comprises a first introducer having distal and proximal ends. The introducer is advantageously configured to be delivered into the anatomical target, such as especially the mitral valve area in or near a mitral plane. In addition, the arrangement comprises also second introducer having distal and proximal ends. The first introducer is an outer introducer and the second introducer is configured to be operated inside and guided by said first introducer. When the first introducer is delivered in or near the mitral plane, the angle of the introducer and thus also the implant is very optimal so that the introducer and thus also the implant will follow the curvature and anatomical shapes of annulus and heart. Thus, no steep curves for the introducers or implants are needed, whereupon the additional introducers and implant are more easily to delivered. This is because the steep curves will increase friction between the introducers as well as inner introducer and the implant. In particularly the friction might be problem, when the implant with memory property is tried to deliver by the curves and if the memory property of the implant will already start to affect.

According to the invention at least a portion, advantageously in a distal portion, of the first introducer is configured to take a first curved shape. In addition, at least a portion, advantageously in a distal portion, of the second introducer is configured to take a second curved shape. Advantageously said first and second curved shapes are concentric curved shapes so that all the curves are curved in the same hand direction and thus form a helical loop structure.

The first introducer is advantageously configured to take said first curved shape when said first introducer is delivered towards or into said anatomical target position. The second introducer is configured to take said second curved shape to the same hand direction as the first curved shape of the first introducer when said second introducer is introduced from the distal end portion of the first introducer. Also, a third introducer can be used with same curvature direction (concentric) and so that it takes a third curved shape to the same hand direction as the previous first and second ones. The curved shape can be taken for example so that there is an operating wire (or the like) arranged to elongate between the proximal and distal ends of the introducer and along a side to which said curved shape is to be provided, whereupon when the operating wire is tightened advantageously from the proximal end of the introducer, it will cause the introducer in question to bend to that direction. There is advantageously a flexible portion arranged inside the curve and into a casing of the introducer so that when the operating wire is tightened, said introducer is caused to take said curved shape at the point of said flexible portion and to said direction said flexible portion locates. The flexible portion can be for example a cutting, such as for example a laser cutting, but also other techniques can be used, such as material weakening, like thinning the wall of the introducer inside the curve. According to embodiment also memory materials can be used.

The concentric way to deliver the introducers has advantages, namely when the introducers and implant is delivered concentric or so that the introducers and implant have a similar curvature direction, there is no need to change the direction of concentric movement of the introducers and implant, which again decreases possible friction or other resistance.

Further, according to an embodiment the portion of the first and/or second introducer taking said curved shape is also configured to form an angle essentially perpendicular to said first and second curved shape directions or to the mitral plane. Thus, the first and second curved shapes turn the introducers concentrically in one plane or circularly, but in addition to this the portion of the first and/or second introducer forms the angle in another perpendicular plane so downwards. Therefore, when the introducer is delivered for example to the left ventricle, the introducer takes a helical loop form due to said first and second curved shapes. In addition to this the distal end portion of the first introducer takes the angle downwards so towards the leaflets, whereupon it alleviates the delivering of the second introducer so that the distal end of the second introducer is pointed towards the leaflets, or the gap between the leaflets, already when the distal end of the second introducer is delivered from the distal end of the first introducer. In addition, also the distal end portion of the second introducer takes the angle downwards so towards the gap of the leaflets, whereupon it is easy to deliver the second or additional introducers between the leaflets and into the opposite side (underside) the annulus.

According to embodiment, a cross-section of the second and/or third introducer is expandable. For example, at least portion of the housing of the introducer can be made expansible, such as made for example of flexible or stretchable material. This allows to deliver a bigger implant, for instance, and especially if the previous introducers are retracted before delivering the implant, whereupon the expandable introducer can take the space of the previous introducers, but still without overtaking any extra space.

As an example, the anatomical target position is a left atrium or left ventricle or an annulus area of a mitral valve. In this case the first introducer is advantageously delivered into a first side of the annulus of the mitral valve and essentially in the mitral plane or in an angle less than in relation to the mitral plane. The second (or further) introducer is then delivered to the second side of the annulus of the mitral valve between leaflets said second side of the annulus being opposite to said first side.

In addition, the invention relates to a method for introducing an object, such as an implant or medicament, into an anatomical target position, wherein in the method
- a first introducer is delivered towards or into the anatomical target position, where at least a portion of first introducer takes a first curved shape,
- a second introducer is delivered towards or into the anatomical target position inside said first introducer, where at least a portion of the second introducer takes a second curved shape when said second introducer is introduced from a distal end of the first introducer,
wherein said first and second curved shapes are concentric curved shapes.

The present invention offers advantages over the known prior art, such as an easy, safe, precise and time saving manner to reliable delivering the implant to the anatomical target position such as to the annulus of the valve. In addition, the present invention provides for a compact arrangement for delivering the implant. The compact medical device allows minimally invasive procedure. Furthermore, when using the compact catheter-operated medical device, risks for having any medical drawbacks or symptoms are much lower than e.g. in the traditional open-heart operation. Also, the patient recovery process is much faster.

The exemplary embodiments presented in this text are not to be interpreted to pose limitations to the applicability of the appended claims. The verb "to comprise" is used in this text as an open limitation that does not exclude the existence of also un-recited features. The features recited in depending claims are mutually freely combinable unless otherwise explicitly stated.

The novel features which are considered as characteristic of the invention are set forth in particular in the appended claims. The invention itself, however, both as to its construction and its method of operation, together with additional objects and advantages thereof, will be best understood from the following description of specific example embodiments when read in connection with the accompanying drawings.

### BRIEF DESCRIPTION OF THE DRAWINGS

Next the invention will be described in greater detail with reference to exemplary embodiments in accordance with the accompanying drawings, in which:
- Figures 1A-1B: illustrate schematically a portion of a heart and mitral valve,
- Figure 2: illustrates an exemplary implant,
- Figures 3-13: illustrate a medical arrangement for introducing an implant or medicament into an anatomical target position according to advantageous embodiments of the invention.

### DETAILED DESCRIPTION

Figures 1A-1B and 2 are already discussed in more details in connection with the background of the invention portion above.

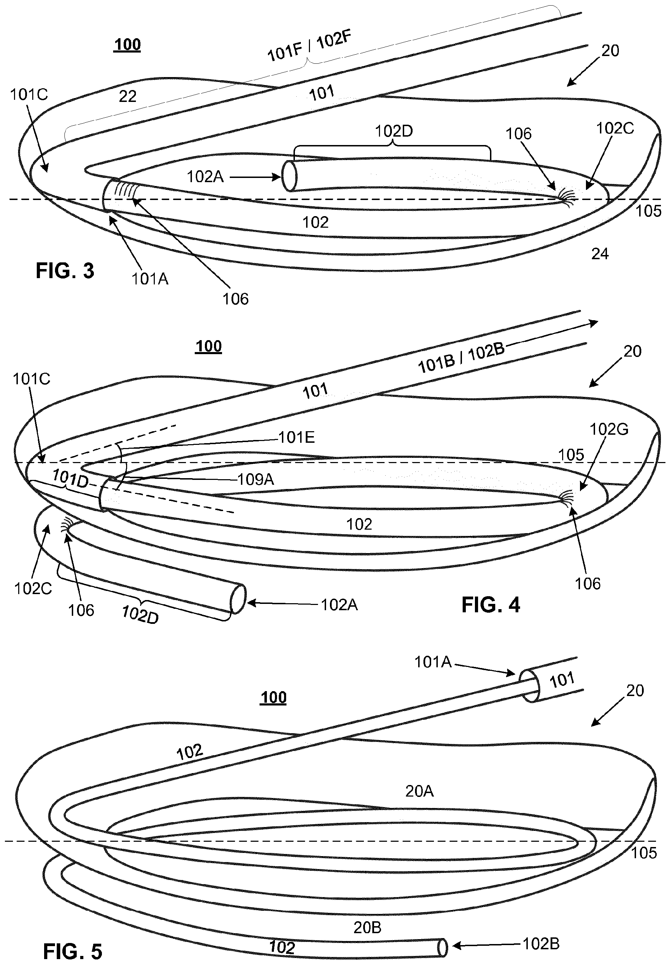

Figures 3-13 illustrate a medical arrangement 100 for introducing an implant 110 into an anatomical target position according to advantageous embodiments of the invention, where the medical arrangement 100 comprises a first introducer 101 having distal and proximal ends 101A, 101B. The introducer is configured to be delivered into the mitral valve area in a mitral plane 105 or in an angle 101E less than 45° and more advantageously less than 30° in relation to the mitral plane 105. In addition, the arrangement comprises also second introducer 102 having distal and proximal ends 102A, 102B.

The first introducer 101 is delivered first in a straightened configuration 101F until the distal end 101A of the first introducer 101 reaches the anatomical target position, whereupon the distal portion is configured to take the first curved shape 101C, advantageously following the anatomical shapes of the anatomical target position. It is to be noted that the first introducer 101 does not typically go further, but after this the second introducer 102 is delivered inside the first introducer 101 in a straightened configuration 102F. The second introducer 102 follows the shapes of the first introducer 101 until it comes out from the distal end 101A of the first introducer 101, after which the second introducer 102 is still delivered further until the distal portion of the second introducer 102 is configured to take the second curved shape 102C, as can be seen in Figure 3.

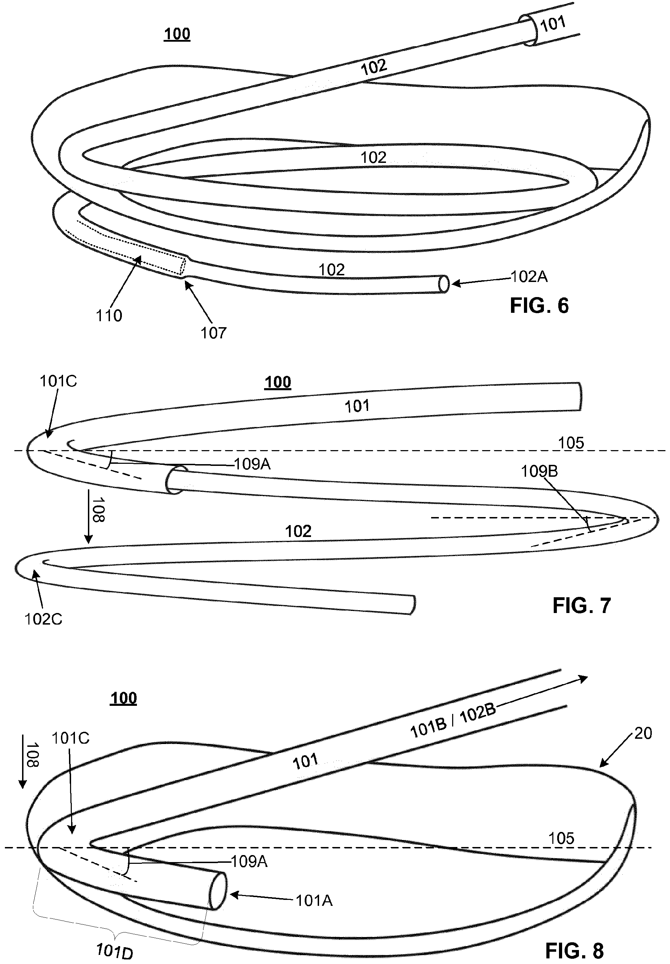

According to an example the second introducer 102 can still be delivered further until the distal portion of the second introducer 102 is configured to take the additional curved shape 102G, as can be seen in Figure 4. The additional curved shape 102G locates to the direction of the proximal end 102B from the second curved shape 102C. However, it is to be noted that the additional curved shape 102G is optional, for example if third or more introducers are used, as is illustrated in Figures 8-12.

Advantageously the second introducer 102 (possibly also the first introducer 101) comprises a flexible portion 106 so that said introducer 101, 102 takes said curved shape 101C, 102C at the point of said flexible portion 106 to the direction where the flexible portion 106 locates. The flexible portion 106 can be a cutting, in particularly a laser cutting, for example or achieved by material weakening. The flexible portion 106 is arranged into a casing of the introducer 101, 102 and so that it is left inside the curve when the introducer takes said curve shape.

It is to be noted that the first and second curved shapes turn the first and second introducers 101, 102 concentrically essentially in the mitral plane 105. However, as can be seen in Figures 3, 4 and 7, for example, the first and second introducers 101, 102 are also configured to tilt or bank downwards 108 and thereby form an angle 109A, 109B also downwards 108. Therefore, when the introducers are delivered for example to the left ventricle, the introducers take a helical loop form due to said first and second and additional curved shapes 101C, 102C, 102G, but in addition to this the distal end portion of the first introducer 101, and advantageously also the second introducer 102 takes the angle downwards 108, whereupon at least the second (or third, if used) introducer 102 can be delivered to the opposite side of the annulus and so that the introducers still follow smoothly the shapes of the anatomical target position.

As can be seen in Figures 5-6, at least the distal portion 101A (or even the whole introducer) of the first introducer 101 is retracted before the implant 110 is introduced into the anatomical target position 20 via the second introducer 102, whereupon more space can be arranged for delivering of the implant, especially if the second introducer is expandable 107. The distal portion 101A of the first introducer 101 is retracted however advantageously after the second introducer 102 is delivered into the anatomical target position 20. If the third introducer 103 is used, as is the case in Figures 8-12, then at least the distal portion 102A of the second introducer 102 is advantageously retracted before the implant 110 is introduced into the anatomical target position 20 and also advantageously after said third introducer 103 is delivered into said anatomical target position 20. In this case also at least the first introducer is retracted.

By this a bigger implant can be delivered without any needs for bigger introducers, namely because when the first introducer 101 is retracted and the second introducer is expandable, the cross-section 107 of the second introducer 102 can expand and take the space needed for the implant. The second introducer can be manufactured for example of flexible or stretchable material. It is to be understood that also the third introducer 103 can be expandable in the cases where the third introducer 103 is used.

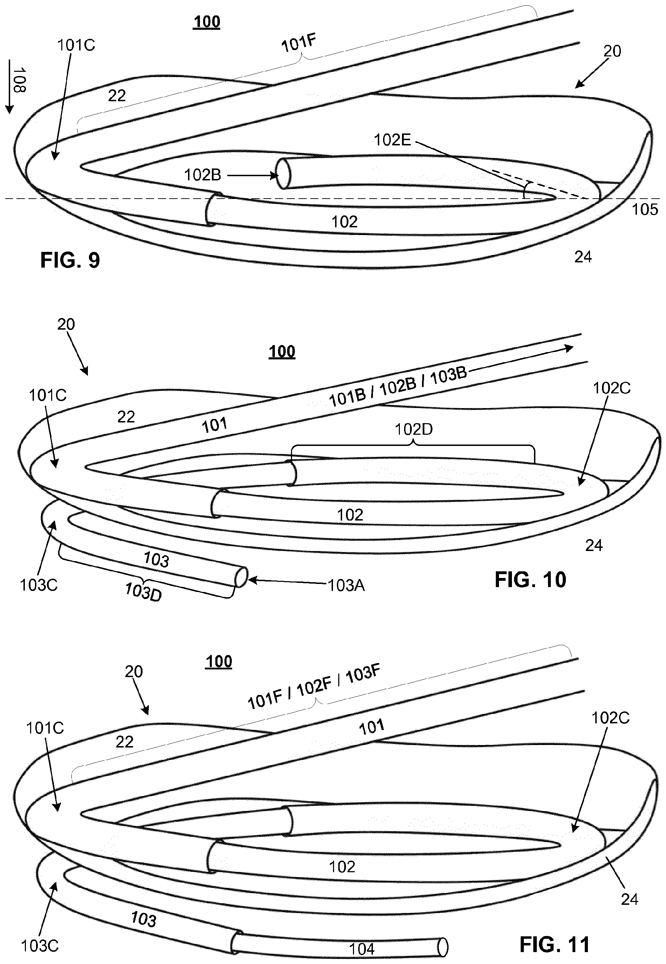

Figures 8-12 illustrate the medical arrangement 100 with third introducer having distal and proximal ends 103A, 103B, where in Fig. 8 the first introducer 101 is delivered so that it takes the first curve shape 101C and the first angle 109A to the downward 108. Next the second introducer 102 is delivered inside the first introducer 101 and from the distal end 101A of the first introducer 101 so that it takes the second curve shape 102C and the second angle 109B to the downward direction 108, as is the case in Fig. 9. Then the third introducer 103 is delivered inside the first and second introducers 101, 102 and from the distal end 102A of the second introducer 102, as can be seen in Figure 10. It is to be noted that the third introducer 103 does not advantageously take any angle 109B to the downward direction 108, because advantageously the third introducer is already delivered to the opposite side of the annulus (between the leaflets) and there is no need to go downwards anymore. Thus, it is advantageous that the third (or any additional) introducer 103, 104 will follow the anatomical target environment, such as the annulus essentially in the mitral plane 105.

However, the third introducer 103 is configured to take the third curved shape 103C to the same hand direction as the first and second curved shapes 101C, 102C of the first and second introducers 101, 102 when the third introducer 103 is introduced from the distal end portion 102A of the second introducer 102 so that said first, second and third curved shapes 101C, 102C, 103C are concentric curved shapes.

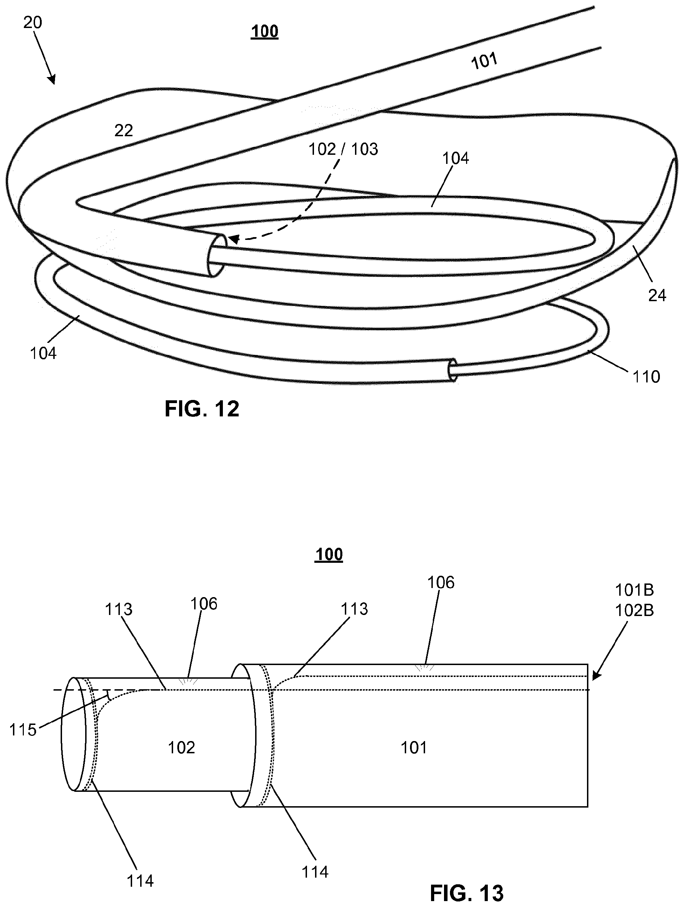

As is the case also with two introducers in Figures 3-7, the first introducer 101 is delivered first and said second introducer 102 is delivered secondly and then the third introducer 103 last. As can be seen in Figure 11, there might be also still additional introducers 104, where the operational principle is analogous with three introducers. In addition, also in this embodiment at least the distal portion 102A of the second introducer 102 is retracted before the implant 110 is introduced into the anatomical target position 20, but anyway after the third introducer 103 is introduced into the anatomical target position. This is for protecting the tissue of the anatomical target position and environment, when the third introducer can be delivered inside the second introducer as far as possible. Same naturally applies to other situations, where for example at least the distal end 101A of the first introducer 101 is retracted after introduction of the third introducer 103 and so on. Figure 12 illustrates an example with four introducers 101-104, where the second and third introducers 102, 103 are retracted and the additional introducer 104 is left to protect the tissue about the delivering of the implant 110. It is to be understood that the additional introducer 104 is illustrated in Fig. 12 so that it is already retracted a certain length towards the proximal end so that the implant delivered can be seen better. In addition, it is to be noted that also the first introducer could have been retracted.

The introducers 101, 102, 103 comprises advantageously also third portions 101D, 102D, 103D between the distal ends 101C, 102C, 103C and said distal portions taking said curved shapes 101C, 102C, 103C. The third portions 101D, 102D, 103D have different curvature radius than the portions taking said curved shapes 101C, 102C, 103C. In some case the third portion 101D, 102D, 103D may be even essentially a direct portion. This will guide the next inner introducer 102, 103 or the implant 110 to take better orientation and direction in relation to the surrounding anatomical environment, because in this way the distal end 102A, 103B, 110B of the outcoming inner introducer 102, 103 or the implant 110 will follow better the shapes of the anatomical target position, i.e. otherwise it will easily hit the tissue of the anatomical environment, such as the annulus.

Figure 13 illustrates an example of the first and second introducers 101,102 how to control the formation of the curved shapes 101C, 102C. The introducer may for example comprise an operating wire 113 (or the like) arranged to elongate between the proximal and distal ends of the introducer and along a side to which said curved shape is to be provided so advantageously the same side where the flexible portion 106 is provided.

The distal ends 101A, 102A of the first and second introducers 101, 102 comprise advantageously a reinforcement ring 114 to which said the operating wire 113 is coupled with. Thus, when the operating wire 113 is tightened advantageously from the proximal end of the introducer 101B, 102B, it will cause the introducer 101, 102 in question to bend to that direction. There flexible portion is arranged inside the curve and into a casing of the introducer so that when the operating wire is tightened, said introducer is caused to take said curved shape at the point of said flexible portion and to said direction said flexible portion locates. The flexible portion can be for example a cutting, such as for example a laser cutting, but also other techniques can be used, such as material weakening, like thinning the wall of the introducer inside the curve. According to embodiment also memory materials can be used.

In addition, it is to be noted that when the operating wire 113 is coupled to the reinforcement ring 114 in an angle 115, the tightening of the operating wire 113 will also tilt or bank the distal end 101A, 102A of the introducer in question to the downward direction 108, as depicted in Figures 3, 4 and 7, for example.

The invention has been explained above with reference to the aforementioned embodiments, and several advantages of the invention have been demonstrated.

In addition, it is to be noted that even if the implant is described in this document as an example to be delivered, also other kinds of object can be delivered according to the invention, such as medicaments, for example. Furthermore, even if the heart is described in many embodiments, it is to be understood that the heart is only an example of the anatomical target.

## Claims

1. A medical arrangement (100) configured to introduce an annuloplasty device (110) from a distal end of the arrangement into an anatomical target position (20), the medical arrangement comprising
- a first introducer (101) having distal and proximal ends (101A, 101B), and
- a second introducer (102) having distal and proximal ends (102A, 102B), wherein
- said first introducer (101) is an outer introducer, and said second introducer is configured to be operated inside and guided by said first introducer, and
- at least a portion of the first introducer (101) is configured to take a first curved shape (101C), and at least a portion of the second introducer (102) is configured to take a second curved shape (102C), wherein said first and second curved shapes (101C, 102C) are concentric curved shapes (101C, 102C),
**characterised in that**
- said first introducer (101) is configured to take said first curved shape (101C) when said first introducer is delivered towards or into said anatomical target position, said second introducer (102) is configured to take said second curved shape (102C) to the same hand direction as the first curved shape (101C) of the first introducer (101) when said second introducer (102) is introduced from the distal end portion (101A) of the first introducer (101),
- said anatomical target position is a left atrium or left ventricle or an annulus area of a mitral valve (18), whereupon said first introducer (101) is configured to be delivered into a first side (20A) of the annulus (20) of the mitral valve essentially in a mitral plane (105) on in an angle less than 45° and more advantageously less than 30° in relation to the mitral plane (105), and
- said portion of the first and second introducer (101, 102) taking said curved shape (101C, 102C) is also configured to form an angle (109A, 109B) in a direction (108) essentially perpendicular to the mitral plane (105), wherein the second introducer is configured to be delivered between leaflets (22, 24) to the second side (20B) of the annulus of the mitral valve said second side of the annulus being opposite to said first side.

2. An arrangement of claim 1, wherein said arrangement comprises additionally a third introducer (103) having distal and proximal ends (103A, 103B), wherein said third introducer (103) is an inner introducer and said second introducer is configured to be operated between said first and third introducer, and wherein said third introducer (103) is configured to take said third curved shape (103C) to the same hand direction as the first and second curved shapes of the first and second introducers (101, 102) when said third introducer (103) is introduced from the distal end portion (102A) of the second introducer (102).

3. An arrangement of any previous claims, wherein said portion of the introducer (101, 102, 103) taking said curved shape (101C, 102C, 103C) is a distal portion of said introducer (101, 102, 103), said distal portion locating between the distal end (101C, 102C, 103C) and proximal end (101A, 102A, 103A) of the introducer (101, 102, 103).

4. An arrangement of claim 3, wherein the introducer (101, 102, 103) comprises a third portion (101D, 102D, 103D) between the distal end (101C, 102C, 103C) and said distal portion taking said taking said curved shape (101C, 102C, 103C), wherein said third portion (101D, 102D, 103D) has a different curvature radius than said portion taking said taking said curved shape (101C, 102C, 103C) or wherein said third portion (101D, 102D, 103D) is essentially a direct portion (101D, 102D, 103D).

5. An arrangement of any previous claims, wherein said second introducer (102) is configured to take at least one additional curved shape (102G), where said additional curved shape (102G) is configured to be located between said second curved shape (102C) and the proximal end (102B).

6. An arrangement of any previous claims, wherein said first introducer (101) is a catheter, in particularly an outer steerable catheter (101), said second introducer (102) is a catheter, in particularly a steerable catheter (102), and said third introducer (103) of claim 2 is a catheter, in particularly an inner steerable catheter (103).

7. An arrangement of any previous claims, wherein said first introducer (101) is configured to be delivered first, and said second introducer (102) is configured to be delivered second and said third introducer (103) of claim 2 is configured to be delivered third.

8. An arrangement of any previous claims, wherein the distal portion (101A) of said first introducer (101) is configured to be retracted before said implant (110) is introduced into said anatomical target position (20) via the second introducer and/or after said second introducer (102) is introduced into said anatomical target position (20); and/or wherein the distal portion (102A) of said second introducer (102) is configured to be retracted before said implant (110) is introduced into said anatomical target position (20) and/or after said third introducer (103) of claim 2 is introduced into said anatomical target position (20).

9. An arrangement of any previous claims, wherein a cross-section of said second introducer (102) or third introducer (103) of claim 2 is expandable and at least portion of the housing of the introducer is expansible (107).

10. An arrangement of claim 2, wherein said third introducer (103) of claim 2 is configured to be delivered to a second side (20B) of the annulus (20) of the mitral valve between leaflets (22, 24), said second side of the annulus being opposite to said first side.

11. An arrangement of any previous claims, wherein the second introducer (102) is configured to be introduced from the distal end (101A) of the first introducer (101) and wherein at least a distal end (102A) of the second introducer (102) is configured to be introduced from the distal end (101A) of the first introducer (101) before the third introducer (103) of claim 2 when used.

12. An arrangement of claim 2, wherein the third introducer (103) is configured to be introduced from the distal end (102A) of the second introducer (103) before introducing said implant.

13. An arrangement of any previous claims, wherein said second curved shape of said second introducer (102) has a first shape capable of being delivered in a straightened configuration (102F) through said first introducer (101) and configured to be activated to said at least second curved shape (102C) within or near the anatomical target position (20); and/or wherein said third curved shape of said third introducer (103) of claim 2 has a first shape capable of being delivered in a straightened configuration (103F) through said first introducer (101) and configured to be activated to said at least third curved shape (103C) within or near the anatomical target position (20).

14. An arrangement of any previous claims, wherein said arrangement is configured to deliver said object (110) inside the second introducer (102) or third introducer (103) of claim 2 into or towards said anatomical target position (20) after at least the distal end (102A, 103A) of the second introducer (102) or third introducer (103) of claim 2 is introduced from the distal end (101A, 102A) of the first or second introducer (101, 102).

15. An arrangement of any previous claims, wherein at least one introducer (101, 102, 103) comprises a flexible portion (106), such as a cutting, in particularly a laser cutting or material weakening, arranged inside the curve and into a casing of the introducer (101, 102, 103) so that said introducer (101, 102, 103) is configured to take said curved shape (101C, 102C, 103C) at the point of said flexible portion (106) and to said direction said flexible portion (106) locates.

## Patentansprüche

1. Medizinische Anordnung (100), die so konfiguriert ist, dass sie eine Annuloplastievorrichtung (110) von einem distalen Ende der Anordnung in eine anatomische Zielposition (20) einführt, wobei die medizinische Anordnung Folgendes umfasst
- eine erste Einführvorrichtung (101) mit einem distalen und einem proximalen Ende (101A, 101B), und
- eine zweite Einführvorrichtung (102) mit einem distalen und einem proximalen Ende (102A, 102B), wobei
- die erste Einführvorrichtung (101) eine äußere Einführvorrichtung ist und die zweite Einführvorrichtung so konfiguriert ist, dass sie innerhalb der ersten Einführvorrichtung betrieben und von dieser geführt wird, und
- mindestens ein Teil der ersten Einführvorrichtung (101) so konfiguriert ist, dass sie eine erste gekrümmte Form (101C) annimmt, und mindestens ein Teil der zweiten Einführvorrichtung (102) so konfiguriert ist, dass sie eine zweite gekrümmte Form (102C) annimmt, wobei die erste und zweite gekrümmte Form (101C, 102C) konzentrische gekrümmte Formen (101C, 102C) sind,
**dadurch gekennzeichnet, dass**
- die erste Einführvorrichtung (101) so konfiguriert ist, dass sie die erste gekrümmte Form (101C) annimmt, wenn die erste Einführvorrichtung zu der anatomischen Zielposition hin oder in diese hinein geführt wird, die zweite Einführvorrichtung (102) so konfiguriert ist, dass sie die zweite gekrümmte Form (102C) in der gleichen Handrichtung wie die erste gekrümmte Form (101C) der ersten Einführvorrichtung (101) annimmt, wenn die zweite Einführvorrichtung (102) von dem distalen Endabschnitt (101A) der ersten Einführvorrichtung (101) eingeführt wird,
- die anatomische Zielposition ein linker Herzvorhof oder ein linker Ventrikel oder ein Anulusbereich einer Mitralklappe (18) ist, woraufhin die erste Einführvorrichtung (101) so konfiguriert ist, dass sie in eine erste Seite (20A) des Anulus (20) der Mitralklappe im Wesentlichen in einer Mitralebene (105) unter einem Winkel von weniger als 45° und vorteilhafterweise weniger als 30° in Bezug auf die Mitralebene (105) zugeführt wird, und
- der Abschnitt der ersten und zweiten Einführvorrichtung (101, 102), der die gekrümmte Form (101C, 102C) annimmt, auch so konfiguriert ist, dass er einen Winkel (109A, 109B) in einer Richtung (108) bildet, die im Wesentlichen senkrecht zur Mitralebene (105) ist, wobei die zweite Einführvorrichtung so konfiguriert ist, dass sie zwischen Segelklappen (22, 24) zur zweiten Seite (20B) des Anulus der Mitralklappe zugeführt wird, wobei die zweite Seite des Anulus der ersten Seite gegenüberliegt.

2. Anordnung nach Anspruch 1, wobei die Anordnung zusätzlich eine dritte Einführvorrichtung (103) mit distalen und proximalen Enden (103A, 103B) umfasst, wobei die dritte Einführvorrichtung (103) eine innere Einführvorrichtung ist und die zweite Einführvorrichtung so konfiguriert ist, dass sie zwischen der ersten und der dritten Einführvorrichtung betrieben wird, und wobei die dritte Einführvorrichtung (103) so konfiguriert ist, dass sie die dritte gekrümmte Form (103C) in dieselbe Handrichtung wie die erste und zweite gekrümmte Form der ersten und zweiten Einführvorrichtung (101, 102) bringt, wenn die dritte Einführvorrichtung (103) vom distalen Endabschnitt (102A) der zweiten Einführvorrichtung (102) eingeführt wird.

3. Eine Anordnung nach einem der vorhergehenden Ansprüche, wobei der Abschnitt der Einführvorrichtung (101, 102, 103), der die gekrümmte Form (101C, 102C, 103C) annimmt, ein distaler Abschnitt der Einführvorrichtung (101, 102, 103) ist, wobei der distale Abschnitt zwischen dem distalen Ende (101C, 102C, 103C) und dem proximalen Ende (101A, 102A, 103A) der Einführvorrichtung (101, 102, 103) angeordnet ist.

4. Eine Anordnung nach Anspruch 3, wobei die Einführvorrichtung (101, 102, 103) einen dritten Abschnitt (101D, 102D, 103D) zwischen dem distalen Ende (101C, 102C, 103C) und dem distalen Abschnitt umfasst, der die gekrümmte Form (101C, 102C, 103C) annimmt, wobei der dritte Abschnitt (101D, 102D, 103D) einen anderen Krümmungsradius aufweist als der die gekrümmte Form annehmende Abschnitt (101C, 102C, 103C) oder wobei der dritte Abschnitt (101D, 102D, 103D) im Wesentlichen ein direkter Abschnitt (101D, 102D, 103D) ist.

5. Eine Anordnung nach einem der vorhergehenden Ansprüche, wobei die zweite Einführvorrichtung (102) so konfiguriert ist, dass sie mindestens eine zusätzliche gekrümmte Form (102G) annimmt, wobei die zusätzliche gekrümmte Form (102G) so konfiguriert ist, dass sie zwischen der zweiten gekrümmten Form (102C) und dem proximalen Ende (102B) angeordnet ist.

6. Eine Anordnung nach einem der vorhergehenden Ansprüche, wobei die erste Einführvorrichtung (101) ein Katheter, insbesondere ein äußerer steuerbarer Katheter (101) ist, die zweite Einführvorrichtung (102) ein Katheter, insbesondere ein steuerbarer Katheter (102) ist, und die dritte Einführvorrichtung (103) nach Anspruch 2 ein Katheter, insbesondere ein innerer steuerbarer Katheter (103) ist.

7. Eine Anordnung nach einem der vorhergehenden Ansprüche, wobei die erste Einführvorrichtung (101) so ausgebildet ist, dass sie als erste zugeführt wird, und die zweite Einführvorrichtung (102) so ausgebildet ist, dass sie als zweite zugeführt wird, und die dritte Einführvorrichtung (103) nach Anspruch 2 so ausgebildet ist, dass sie als dritte zugeführt wird.

8. Eine Anordnung nach einem der vorhergehenden Ansprüche, wobei der distale Abschnitt (101A) der ersten Einführvorrichtung (101) so konfiguriert ist, dass er zurückgezogen wird, bevor das Implantat (110) über die zweite Einführvorrichtung in die anatomische Zielposition (20) eingeführt wird und/oder nachdem die zweite Einführvorrichtung (102) in die anatomische Zielposition (20) eingeführt wurde; und/oder wobei der distale Abschnitt (102A) der zweiten Einführvorrichtung (102) so konfiguriert ist, dass er zurückgezogen wird, bevor das Implantat (110) in die anatomische Zielposition (20) eingeführt wird und/oder nachdem die dritte Einführvorrichtung (103) nach Anspruch 2 in die anatomische Zielposition (20) eingeführt wurde.

9. Eine Anordnung nach einem der vorhergehenden Ansprüche, wobei ein Querschnitt der zweiten Einführvorrichtung (102) oder der dritten Einführvorrichtung (103) nach Anspruch 2 dehnbar ist und zumindest ein Teil des Gehäuses der Einführvorrichtung dehnbar ist (107).

10. Eine Anordnung nach Anspruch 2, wobei die dritte Einführvorrichtung (103) nach Anspruch 2 so konfiguriert ist, dass sie an einer zweiten Seite (20B) des Anulus (20) der Mitralklappe zwischen den Segelklappen (22, 24) zugeführt wird, wobei die zweite Seite des Anulus der ersten Seite gegenüberliegt.

11. Eine Anordnung nach einem der vorhergehenden Ansprüche, wobei die zweite Einführvorrichtung (102) so konfiguriert ist, dass sie vom distalen Ende (101 A) der ersten Einführvorrichtung (101) aus eingeführt wird, und wobei mindestens ein distales Ende (102A) der zweiten Einführvorrichtung (102) so konfiguriert ist, dass es vom distalen Ende (101 A) der ersten Einführvorrichtung (101) noch vor der dritten Einführvorrichtung (103) nach Anspruch 2 während des Verwendens, eingeführt wird.

12. Eine Anordnung nach Anspruch 2, wobei die dritte Einführvorrichtung (103) so konfiguriert ist, dass sie vom distalen Ende (102A) der zweiten Einführvorrichtung (103) aus eingeführt wird, bevor das Implantat eingeführt wird.

13. Eine Anordnung nach einem der vorhergehenden Ansprüche, wobei die zweite gekrümmte Form der zweiten Einführvorrichtung (102) eine erste Form aufweist, die in einer geraden Konfiguration (102F) durch die erste Einführvorrichtung (101) zugeführt werden kann und so konfiguriert ist, dass sie in die mindestens zweite gekrümmte Form (102C) innerhalb oder in der Nähe der anatomischen Zielposition (20) aktiviert wird; und/oder wobei die dritte gekrümmte Form der dritten Einführvorrichtung (103) nach Anspruch 2 eine erste Form hat, die in einer geraden Konfiguration (103F) durch die erste Einführvorrichtung (101) zugeführt werden kann und so konfiguriert ist, dass sie zu der mindestens dritten gekrümmten Form (103C) innerhalb oder nahe der anatomischen Zielposition (20) aktiviert wird.

14. Eine Anordnung nach einem der vorhergehenden Ansprüche, wobei die Anordnung so konfiguriert ist, dass sie das Objekt (110) innerhalb der zweiten Einführvorrichtung (102) oder der dritten Einführvorrichtung (103) nach Anspruch 2 in die oder in Richtung der anatomischen Zielposition (20) zuführt, nachdem zumindest das distale Ende (102A, 103A) der zweiten Einführvorrichtung (102) oder der dritten Einführvorrichtung (103) nach Anspruch 2 von dem distalen Ende (101A, 102A) der ersten oder zweiten Einführvorrichtung (101, 102) eingeführt wurde.

15. Eine Anordnung nach einem der vorhergehenden Ansprüche, wobei mindestens eine Einführvorrichtung (101, 102, 103) einen flexiblen Abschnitt (106), wie einen Schnitt, insbesondere einen Laserschnitt oder eine Materialschwächung, aufweist, der innerhalb der Krümmung und in einem Gehäuse der Einführvorrichtung (101, 102, 103) angeordnet ist, so dass die Einführvorrichtung (101, 102, 103) so konfiguriert ist, dass sie die gekrümmte Form (101C, 102C, 103C) an der Stelle des flexiblen Abschnitts (106) und in der Richtung annimmt, in der der flexible Abschnitt (106) angeordnet ist.

## Revendications

1. Agencement médical (100) configuré pour introduire un dispositif d'annuloplastie (110) à partir d'une extrémité distale de l'agencement dans une position cible anatomique (20), l'agencement médical comprenant
- un premier introducteur (101) ayant des extrémités distale et proximale (101 A, 101B),
et
- un deuxième introducteur (102) ayant des extrémités distale et proximale (102A, 102B), dans lequel
- ledit premier introducteur (101) est un introducteur externe, et ledit deuxième introducteur est configuré pour fonctionner à l'intérieur et être guidé par ledit premier introducteur, et
- au moins une partie du premier introducteur (101) est configurée pour prendre une première forme incurvée (101C), et au moins une partie du deuxième introducteur (102) est configurée pour prendre une deuxième forme incurvée (102C), dans lequel lesdites première et deuxième formes incurvées (101C, 102C) sont des formes incurvées concentriques (101C, 102C),
**caractérisé en ce que**
- ledit premier introducteur (101) est configuré pour prendre ladite première forme incurvée (101C) lorsque ledit premier introducteur est délivré vers ou dans ladite position cible anatomique, ledit deuxième introducteur (102) est configuré pour prendre ladite deuxième forme incurvée (102C) dans la même direction de la main que la première forme incurvée (101C) du premier introducteur (101) lorsque ledit deuxième introducteur (102) est introduit à partir de la partie d'extrémité distale (101A) du premier introducteur (101),
- ladite position cible anatomique est une oreillette gauche ou un ventricule gauche ou une zone annulaire d'une valvule mitrale (18), après quoi ledit premier introducteur (101) est configuré pour être délivré dans un premier côté (20A) de l'espace annulaire (20) de la valvule mitrale sensiblement dans un plan mitral (105) selon un angle inférieur à 45° et plus avantageusement inférieur à 30° par rapport au plan mitral (105), et
- ladite partie des premier et deuxième introducteurs (101, 102) prenant ladite forme incurvée (101C, 102C) est également configurée pour former un angle (109A, 109B) dans une direction (108) sensiblement perpendiculaire au plan mitral (105), dans lequel le deuxième introducteur est configuré pour être délivré entre des feuillets (22, 24) vers le second côté (20B) de l'espace annulaire de la valvule mitrale, ledit second côté de l'espace annulaire étant opposé audit premier côté.

2. Agencement selon la revendication 1, dans lequel ledit agencement comprend en outre un troisième introducteur (103) ayant des extrémités distale et proximale (103A, 103B), dans lequel ledit troisième introducteur (103) est un introducteur interne et ledit deuxième introducteur est configuré pour fonctionner entre lesdits premier et troisième introducteurs, et dans lequel ledit troisième introducteur (103) est configuré pour prendre ladite troisième forme incurvée (103C) dans la même direction de la main que les première et deuxième formes incurvées des premier et deuxième introducteurs (101, 102) lorsque ledit troisième introducteur (103) est introduit à partir de la partie d'extrémité distale (102A) du deuxième introducteur (102).

3. Agencement selon l'une quelconque des revendications précédentes, dans lequel ladite partie de l'introducteur (101, 102, 103) prenant ladite forme incurvée (101C, 102C, 103C) est une partie distale dudit introducteur (101, 102, 103), ladite partie distale étant située entre l'extrémité distale (101C, 102C, 103C) et l'extrémité proximale (101A, 102A, 103A) de l'introducteur (101, 102, 103).

4. Agencement selon la revendication 3, dans lequel l'introducteur (101, 102, 103) comprend une troisième partie (101D, 102D, 103D) entre l'extrémité distale (101C, 102C, 103C) et ladite partie distale prenant ladite prenant ladite forme incurvée (101C, 102C, 103C), dans lequel ladite troisième partie (101D, 102D, 103D) a un rayon de courbure différent de celui de ladite partie prenant ladite prenant ladite forme incurvée (101C, 102C, 103C) ou dans lequel ladite troisième partie (101D, 102D, 103D) est sensiblement une partie directe (101D, 102D, 103D).

5. Agencement selon l'une quelconque des revendications précédentes, dans lequel ledit deuxième introducteur (102) est configuré pour prendre au moins une forme incurvée supplémentaire (102G), où ladite forme incurvée supplémentaire (102G) est configurée pour être située entre ladite deuxième forme incurvée (102C) et l'extrémité proximale (102B).

6. Agencement selon l'une quelconque des revendications précédentes, dans lequel ledit premier introducteur (101) est un cathéter, en particulier un cathéter externe orientable (101), ledit deuxième introducteur (102) est un cathéter, en particulier un cathéter orientable (102), et ledit troisième introducteur (103) selon la revendication 2 est un cathéter, en particulier un cathéter interne orientable (103) .

7. Agencement selon l'une quelconque des revendications précédentes, dans lequel ledit premier introducteur (101) est configuré pour être délivré en premier, et ledit deuxième introducteur (102) est configuré pour être délivré en deuxième et ledit troisième introducteur (103) selon la revendication 2 est configuré pour être délivré en troisième.

8. Agencement selon l'une quelconque des revendications précédentes, dans lequel la partie distale (101A) dudit premier introducteur (101) est configurée pour être rétractée avant que ledit implant (110) ne soit introduit dans ladite position cible anatomique (20) via le deuxième introducteur et/ou après que ledit deuxième introducteur (102) a été introduit dans ladite position cible anatomique (20) ; et/ou dans lequel la partie distale (102A) dudit deuxième introducteur (102) est configurée pour être rétractée avant que ledit implant (110) ne soit introduit dans ladite position anatomique cible (20) et/ou après que ledit troisième introducteur (103) selon la revendication 2 a été introduit dans ladite position cible anatomique (20).

9. Agencement selon l'une quelconque des revendications précédentes, dans lequel une section transversale dudit deuxième introducteur (102) ou troisième introducteur (103) selon la revendication 2 est extensible et au moins une partie du boîtier de l'introducteur est expansible (107).

10. Agencement selon la revendication 2, dans lequel ledit troisième introducteur (103) selon la revendication 2 est configuré pour être délivré sur un second côté (20B) de l'espace annulaire (20) de la valvule mitrale entre des feuillets (22, 24), ledit second côté de l'espace annulaire étant opposé audit premier côté.

11. Agencement selon l'une quelconque des revendications précédentes, dans lequel le deuxième introducteur (102) est configuré pour être introduit à partir de l'extrémité distale (101A) du premier introducteur (101) et dans lequel au moins une extrémité distale (102A) du deuxième introducteur (102) est configurée pour être introduite à partir de l'extrémité distale (101A) du premier introducteur (101) avant le troisième introducteur (103) selon la revendication 2 lorsqu'il est utilisé.

12. Agencement selon la revendication 2, dans lequel le troisième introducteur (103) est configuré pour être introduit à partir de l'extrémité distale (102A) du deuxième introducteur (103) avant d'introduire ledit implant.

13. Agencement selon l'une quelconque des revendications précédentes, dans lequel ladite deuxième forme incurvée dudit deuxième introducteur (102) a une première forme capable d'être délivrée dans une configuration redressée (102F) à travers ledit premier introducteur (101) et configurée pour être activée à ladite au moins une deuxième forme incurvée (102C) à l'intérieur ou à proximité de la position cible anatomique (20) ; et/ou dans lequel ladite troisième forme incurvée dudit troisième introducteur (103) selon la revendication 2 a une première forme capable d'être délivrée dans une configuration redressée (103F) à travers ledit premier introducteur (101) et configurée pour être activée à ladite au moins troisième forme incurvée (103C) à l'intérieur ou à proximité de la position cible anatomique (20).

14. Agencement selon l'une quelconque des revendications précédentes, dans lequel ledit agencement est configuré pour délivrer ledit objet (110) à l'intérieur du deuxième introducteur (102) ou du troisième introducteur (103) selon la revendication 2 dans ou vers ladite position cible anatomique (20) après qu'au moins l'extrémité distale (102A, 103A) du deuxième introducteur (102) ou du troisième introducteur (103) selon la revendication 2 a été introduite à partir de l'extrémité distale (101A, 102A) du premier ou du deuxième introducteur (101, 102).

15. Agencement selon l'une quelconque des revendications précédentes, dans lequel au moins un introducteur (101, 102, 103) comprend une partie flexible (106), telle qu'une découpe, en particulier une découpe laser ou un affaiblissement de matériau, agencée à l'intérieur de la courbe et dans un carter de l'introducteur (101, 102, 103) de sorte que ledit introducteur (101, 102, 103) est configuré pour prendre ladite forme incurvée (101C, 102C, 103C) au niveau de ladite partie flexible (106) et dans ladite direction dans laquelle ladite partie flexible (106) se situe.
